# FASCICULE DE BREVET EUROPEEN

(11) **EP 1 112 755 B1**
(45) Date de publication et mention de la délivrance du brevet: **21.09.2005**
(21) Numéro de dépôt: 00403657.0
(22) Date de dépôt: 22.12.2000
(51) Int. Cl.: A61N 1/362

(54) **Dispositif médical implantable actif notamment stimulateur cardiaque, défibrillateur et/ou cardioverteur, comprenant des moyens d'élimination des artefacts de détection des événements**
Aktive medizinische implantierbare Vorrichtung, insbesondere Herzstimulator, Defibrillator oder Kardiovertierer, mit Mitteln zur Beseitigung von Artefakten der Erfassung von Ereignissen
Active medical implantable device, in particular pacemaker, defibrillator or cardioverter, having means for eliminating artefacts of detection of events

(30) Priorité: 29.12.1999 FR 9916634
(43) Date de publication de la demande: 04.07.2001
(73) Titulaire: ELA MEDICAL (Société anonyme), 92541 Montrouge (FR)
(72) Inventeur: Bonnet, Jean-Luc, 92120 Montrouge (FR); Henry, Christine, 75014 Paris (FR)
(74) Mandataire: Dupuis-Latour, Dominique

(56) Documents cités:
- EP-A- 0 334 618
- EP-A- 0 813 888
- US-A- 5 718 242

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, tous dispositifs dont le fonctionnement dépend de la détection des signaux cardiaques spontanément produits par le coeur du patient porteur du dispositif pour enregistrer des données, poser un diagnostic ou appliquer une thérapie appropriée.

Il est indispensable que le comportement du dispositif respecte la physiologie du patient ou, à tout le moins, ne crée aucun risque pour lui. Une difficulté rencontrée à cet égard réside dans les problèmes de sur-détection des événements, c'est-à-dire les situations où le dispositif détecte non seulement l'événement proprement dit (onde de dépolarisation de la cavité considérée), mais également un artefact associé à ce même événement et considéré, à tort, comme un autre événement survenu après le premier.

Ces artefacts peuvent être d'origines diverses. Ainsi, lorsque les événements spontanés présentent plusieurs composantes dont certaines surviennent tardivement après la détection, par exemple l'onde de repolarisation ou onde T, cette composante tardive peut être détectée, à tort, comme un nouvel événement spontané distinct du précédent. Il en est de même pour la diaphonie, qui est la détection dans une cavité d'une activité, stimulation ou détection, provenant de l'autre cavité, par exemple et généralement la détection dans le canal auriculaire d'une activité ventriculaire (dépolarisation ou repolarisation).

Diverses techniques ont été proposées pour pallier ces risques de sur-détection, notamment :
- l'application d'un filtrage, analogique ou numérique, pour éliminer notamment les composantes rapides du signal de repolarisation,
- l'application de périodes réfractaires, pour une même cavité ou entre cavités,
- l'adaptation automatique de sensibilité des amplificateurs de détection, ou le contrôle automatique du gain de ces amplificateurs.

Cependant, l'utilisation de ces divers moyens se fait toujours au détriment d'une bonne détection, principalement dans le cas où il n'y a pas de surdétection.

Ainsi, les périodes réfractaires longues permettent de protéger le dispositif contre des détections intempestives, mais elles réduisent la capacité d'écoute du système et risquent de conduire à des "faux négatifs" si elles sont trop longues et laissent passer un événement sans le détecter.

De la même façon, afin d'assurer la détection de la fibrillation ventriculaire (FV), le niveau du signal étant faible, il est nécessaire de rechercher une sensibilité maximale sous peine, ici encore, de ne pas détecter des événements qui auraient dû l'être.

Il existe également des risques de détection de "faux positifs", notamment dans le cas de certains signaux endocavitaires particulièrement longs. Il s'agit généralement de patients présentant des cardiopathies dilatées pour lesquelles la durée des dépolarisations, principalement ventriculaires, est alors accrue.

L'analyse des intervalles de temps séparant les événements successifs détectés (intervalles RR dans le cas des événements ventriculaires) est mise en oeuvre dans divers algorithmes de diagnostic des stimulateurs, comme cela est décrit par exemple dans le EP-A-0 626 182 (Ela Médical), et les doubles détections sont bien évidemment de nature à leurrer ce type d'algorithme et aboutir à des actions inappropriées du stimulateur (ici et dans la suite on entendra par "stimulateur" aussi bien le générateur d'impulsions d'un stimulateur cardiaque *stricto sensu* que celui d'un défibrillateur ou cardioverteur).

Ainsi, pour un patient présentant un rythme cardiaque sinusal normal, la présence de doubles détections se traduirait par une évaluation du rythme moyen à un niveau très supérieur au rythme réel, avec dans ce cas le risque d'application d'une thérapie antitachycardique indésirable (choc de cardioversion ou de défibrillation) dans le cas d'un cardioverteur/défibrillateur. L'application d'un des moyens précédents aurait pour conséquence un risque de non-diagnostic de la FV.

Les systèmes de protection que l'on vient de présenter sont de ce fait incapables de protéger l'appareil contre une double détection du même événement cardiaque.

L'un des buts de l'invention est précisément d'éviter un tel risque de double détection, par des moyens applicables notamment aux situations typiques décrites ci-dessus, sans pour autant réduire la capacité d'écoute du dispositif.

Le point de départ de l'invention réside dans la constatation que :
- d'une part, le doublet résultant d'une "double détection" (c'est-à-dire la détection de l'événement et également celle d'un artefact lié à cet événement), doublet vu à tort par le dispositif comme une succession de deux événements distincts, a lieu généralement précocement dans le cycle, et
- d'autre part, l'intervalle de temps séparant deux doublets successifs est plus long que celui séparant les deux détections du doublet (en d'autres termes, l'intervalle événement-artefact est plus court que l'intervalle artefact-événement suivant).

Cette situation de "double détection" erronée se distingue des situations d'arythmie, où les intervalles sont généralement courts et stables pour des tachycardies, et instables pour des fibrillations, situations révélées par des détections d'événements rapprochés qu'il y a lieu de ne pas confondre avec des doubles détections.

Le principe de base de l'invention consiste à reconnaître une alternance intervalle court/intervalle long révélatrice d'une double détection - devant être prise en compte par les moyens d'analyse du signal du dispositif, notamment lorsque ceux-ci déterminent le rythme cardiaque à partir des détections successives.

Ainsi, si l'on désigne par L un cycle long correspondant au couplage sinusal normal, par C un cycle court (typiquement dû à un artefact) et par M un cycle de durée intermédiaire intervenant après un cycle C, on obtient les relations suivantes dans le cas de la détection erronée d'un signal tardif :
■ durée L ≈ durée C + durée M
■ nombre de cycles C = nombre de cycles M
■ un cycle C est toujours suivi d'un cycle M (motif CM)

Une séquence type décrivant un phénomène de détection tardive sur quelques cycles pourrait être : L L L C M C M C M C M L L .

Plus précisément, le dispositif de l'invention est du type connu comprenant : des moyens de détection des événements spontanés dans une cavité cardiaque, ventriculaire ou auriculaire ; des moyens de mesure des intervalles séparant les événements successifs ainsi détectés ; des moyens d'analyse du rythme cardiaque, opérant en fonction des valeurs d'intervalles ainsi mesurées ; et des moyens d'élimination des doubles détections d'un même événement, lorsqu'un événement est suivi d'un artefact susceptible d'être également détectés.

Selon l'invention, les moyens d'élimination des doubles détections comprennent des moyens aptes à évaluer des intervalles successifs mesurés séparant une série d'événements consécutifs détectés, à y identifier des intervalles courts et des intervalles longs, et considérer qu'il y a double détection en présence d'une alternance d'intervalles courts et d'intervalles longs dans les intervalles successifs.

Dans une mise en oeuvre avantageuse préférentielle, les moyens d'élimination des doubles détections comprennent des moyens d'analyse statistique de la distribution des valeurs desdits intervalles successifs.

Ces derniers moyens peuvent en particulier comprendre : des moyens de classification, aptes à distribuer lesdits intervalles successifs en un histogramme comportant une pluralité de classes correspondant à des plages de valeurs consécutives d'intervalles ; et des moyens aptes à déterminer, à partir du nombre d'intervalles classés dans chaque plage, la présence de deux pics statistiques équivalents dans l'histogramme et, dans ce cas, considérer qu'il y a eu double détection.

En particulier, les moyens d'analyse statistique de la distribution ne déterminent qu'il y a présence de deux pics équivalents que si :
- ces pics sont des pics distincts, par exemple des pics séparés par au moins une classe vide ;
- le pic principal rassemble plus de 40 % du nombre total des intervalles ;
- le pic secondaire rassemble autant d'intervalles que le pic principal.

Un critère supplémentaire peut être mis en oeuvre avec des moyens aptes à évaluer la durée C des cycles courts, la durée L des cycles longs et la durée M des cycles intermédiaires, et à signaler aux moyens d'analyse du rythme cardiaque qu'il y a eu double détection si : L ≈ C + M.

On va maintenant décrire un exemple de mise en oeuvre de l'invention, en référence aux dessins annexés.
La figure 1 est un chronogramme montrant la succession des événements recueillis sur les canaux de détection auriculaire et ventriculaire, dans une situation de double détection.
La figure 2 est un histogramme des intervalles RR dans une situation de double détection avérée.
Les figures 3 à 5 sont des histogrammes des intervalles RR correspondant à des situations dans lesquelles il n'est pas possible de conclure à la présence d'une situation de double détection.

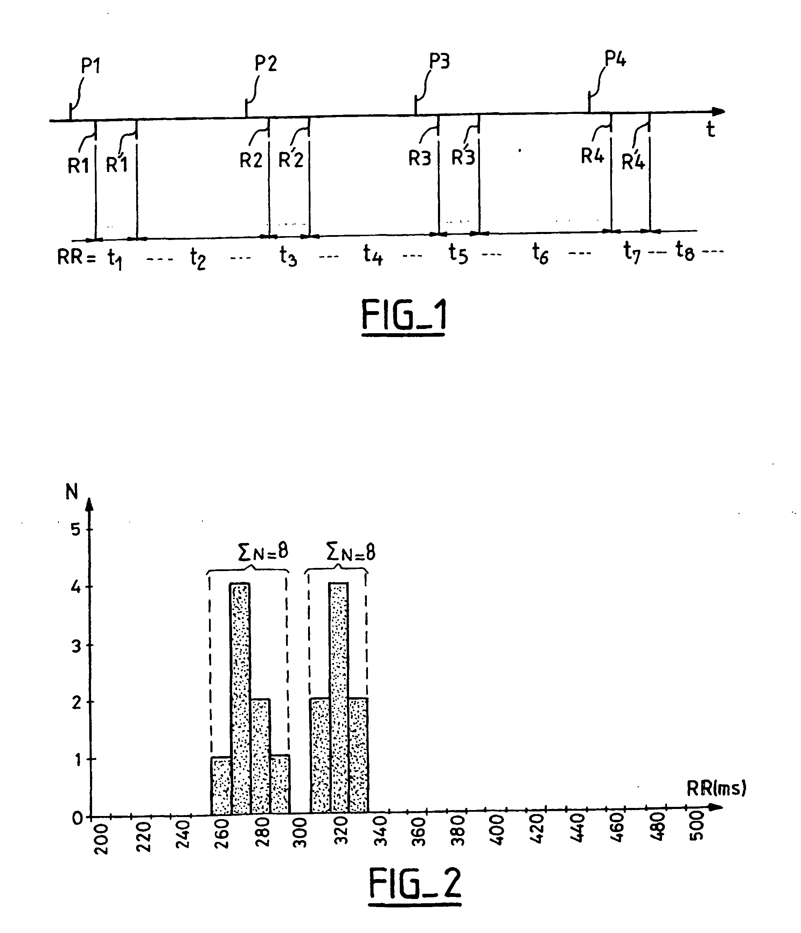

La figure 1 est un chronogramme montrant les signaux cardiaques détectés dans le cas typique d'une double détection. Le complexe PQRST correspondant à une dépolarisation cardiaque se traduit par une détection auriculaire ou onde P (P₁, P₂, P₃ ..., suivie par une détection ventriculaire, sur l'onde R de la composante QRS (R₁, R₂, R₃ ...).

Dans le cas d'une double détection, la détection ventriculaire R₁, R₂, R₃ ... sur le complexe QRS est suivie d'une fausse détection R'₁, R'₂, R'₃ ... constituant un artefact.

Cette fausse détection peut être provoquée notamment par un résidu d'onde T après filtrage, ou bien par une extrasystole systématique sur dépolarisation ventriculaire (bigéminisme, qui est une situation ne devant pas provoquer l'application d'une thérapie).

De façon caractéristique, les intervalles R₁-R'₁, R₂-R'₂, R₃-R'₃ ... séparant la vraie détection de l'artefact sur le même cycle cardiaque sont relativement stables, de l'ordre de 200 ms.

En revanche, les intervalles R'₁-R₂, R'₂-R₃ ... séparant l'artefact de la détection sur le cycle cardiaque suivant peuvent être assez variables et ne sont pas prévisibles, car ils dépendent en fait du rythme sinusal du patient. Ces intervalles peuvent varier typiquement de 400 ms (à l'effort) à 800 ms (au repos), mais ils sont toujours plus longs que les intervalles R₁-R'₁, R₂-R'₂, R₃-R'₃ ... et ce, de façon significative.

C'est une telle situation que, selon l'invention, le dispositif va tenter de détecter afin de reconnaître une situation de double détection.

On se placera dans l'hypothèse où la double détection (présence de R et R' sur un même cycle cardiaque) est quasiment présente en permanence, c'est-à-dire où cette situation est installée par exemple sur 80 % des cycles cardiaques ; dans le cas contraire, s'il ne s'agissait que de l'apparition sporadique de doubles détections, les conséquences de ces dernières serait fortement minimisé par l'effet de moyennage de l'algorithme sur quelques cycles cardiaques et d'analyse de la stabilité des intervalles, sans qu'il soit nécessaire de procéder à une discrimination supplémentaire.

Pour la mise en oeuvre de l'invention, le dispositif comporte, de manière en elle-même connue :
- des moyens de détection des événements cardiaques atriaux et/ou ventriculaires, par exemple des événements ventriculaires R₁, R'₁, R₂, R'₂, R₃, R'₃, R₄, R'₄ ... dans l'exemple illustré, avec au moins une sonde endocavitaire, et des circuits d'amplification et de filtrage du signal recueilli par cette sonde,
- des moyens de mesure des intervalles de temps t₁, t₂, t₃, t₃, t₄, t₅... écoulés entre deux activités successivement détectées,
- des moyens de classification des intervalles cardiaques, typiquement sous forme d'histogrammes tels que ceux illustrés sur les figures 2 à 5.

Ces moyens sont notamment mis en oeuvre par l'algorithme "PARAD" (marque déposée d'ELA Médical), qui est un algorithme de diagnostic des arythmies mis en oeuvre dans des appareils tels que les défibrillateurs DEFENDER d'ELA Médical, algorithme faisant notamment l'objet de la demande EP-A-0 626 182.

Les moyens de classification des intervalles cardiaques mesurés répartissent ceux-ci en un certain nombre de classes ou plages correspondant à des intervalles consécutifs joints et de même largeur, typiquement 10 ms. Tous les intervalles ont le même poids dans l'histogramme (poids unitaire), et celui-ci ne conserve qu'un certain nombre prédéterminé d'intervalles consécutifs, typiquement les 16 derniers intervalles ; à chaque cycle, l'intervalle le plus ancien est retiré alors que le plus récent est inclus dans l'histogramme. Ce dernier est donc remis à jour à chaque cycle et contient l'analyse de la distribution des seize derniers intervalles cardiaques mesurés.

Par souci de simplification, on peut filtrer les intervalles pour lesquels la durée est supérieure à un seuil donné (connu sous le nom de "*cut-off rate*" ou "TDI", *Tachycardia Detection Interval*). Dans le cas contraire, on pourra prévoir un critère de sélection supplémentaire, en ne retenant pour analyse des doubles détections que les cycles pour lesquels est vérifiée la relation : durée L ≈ durée C + durée M (C désignant les cycles courts, L les cycles longs et M les cycles de durée intermédiaire).

A partir des histogrammes ainsi constitués (dont différents exemples sont illustrés sur les figures 2 à 5), le dispositif recherche la présence d'un ou plusieurs pics.

Pour ce faire, le dispositif calcule l'amplitude d'une série de classes, typiquement une série formée de la somme de quatre classes consécutives, et recherche la série présentant la somme ΣN la plus grande à partir de la gauche (c'est-à-dire à partir de la classe des intervalles les plus courts), série qui sera définie comme étant le "pic principal".

Sur la figure 2, ce pic principal est le pic de gauche, correspondant aux quatre classes 260-270 ms, 270-280 ms, 280-290 ms et 290-300 ms, regroupant un total de ΣN = 8 détections.

Le système recherche ensuite de la même manière un autre pic, qui sera appelé "pic secondaire".

Sur la figure 2, ce pic secondaire correspond au pic de droite, regroupe des intervalles dans la série 310-320 ms, 320-330 ms et 330-340 ms, pour un total ΣN = 8 détections.

Comme on le comprendra aisément, dans le cas d'une double détection stable et avérée correspondant au chronogramme de la figure 1, le pic principal correspond aux intervalles courts t₁, t₃, t₅..., tandis que le pic secondaire correspond aux intervalles longs t₂, t₄, t₆ ...

Le système vérifie ensuite si le pic principal et le pic secondaire sont "équivalents", c'est-à-dire s'ils répondent aux différents critères que l'on va exposer ci-dessous. Si les deux pics sont équivalents, le dispositif considère qu'une double détection stable est bien installée, phénomène qui est signalé aux moyens d'analyses du rythme cardiaque pour prise en compte.

Une *première condition* pour que les deux pics, principal et secondaire, soient équivalents est que le pic principal regroupe plus de 40 % des intervalles détectés.

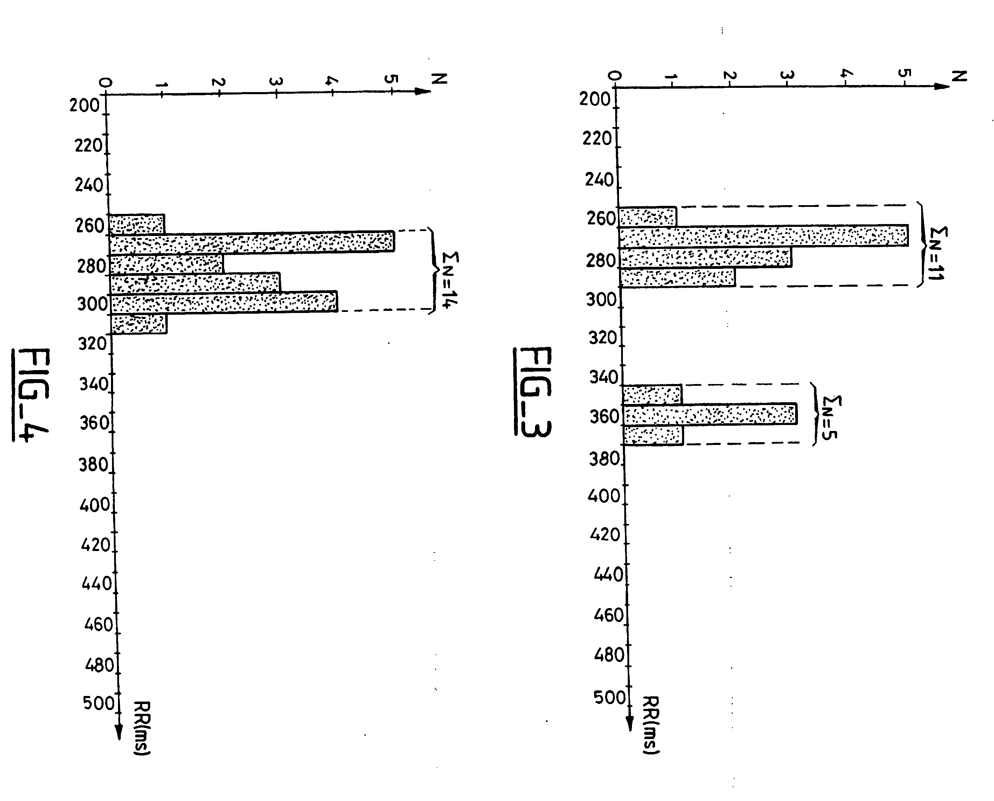

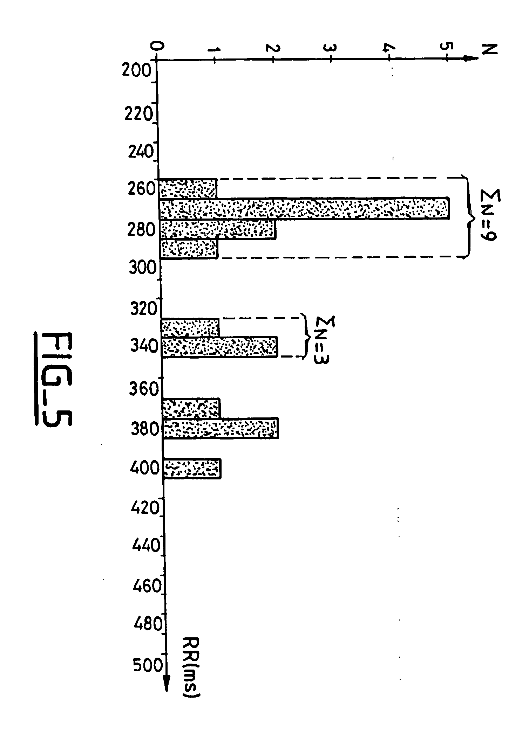

Cette première condition est vérifiée dans le cas de l'histogramme de la figure 2, ainsi que dans celui des figures 3 et 5, mais pas dans celui de la figure 4.

Une *seconde condition* pour que les deux pics soient considérés comme équivalents est que ceux-ci soient des pics disjoints, c'est-à-dire séparés par au moins une classe vide entre les deux séries de valeurs de chaque pic.

Cette condition est vérifiée sur les histogrammes des figure 2 et 3. Elle ne l'est pas sur l'histogramme de la figure 4, où l'on ne trouve pas deux pics distincts mais un seul pic regroupant une très grande majorité des intervalles (dans l'exemple de la figure 4, quatorze intervalles sur seize). Cette situation où les deux pics ne sont pas disjoints correspond très vraisemblablement à une tachycardie, qui devra être diagnostiquée et confirmée par d'autres critères.

Une *troisième condition* est que le pic secondaire regroupe autant de cycles que le pic principal.

Tel est le cas sur l'exemple d'histogramme de la figure 2, où le pic secondaire regroupe huit intervalles sur les seize. Tel n'est pas le cas, en revanche, sur les histogrammes des figure 3 et 5, bien que les deux premières conditions soient vérifiées. Cette dernière situation correspond vraisemblablement à une fibrillation auriculaire, qui ne pourra cependant être diagnostiquée et confirmée que par une analyse basée sur d'autres critères.

Dans tous les cas, si le dispositif ne peut pas conclure à la présence d'une double détection stable, c'est-à-dire si l'une au moins des trois conditions ci-dessus n'est pas respectée, le dispositif poursuit son analyse sur la base d'autres critères, tels que ceux décrits par exemple dans le EP-A-0 626 182 ou le EP-A-0 838 235, qui décrivent divers procédés d'analyse du rythme cardiaque basés principalement sur l'analyse de la stabilité et de l'évolution des intervalles RR et PR.

Il est possible de rajouter un critère sur la position du premier pic (c'est-à-dire celui regroupant les intervalles les plus courts, correspondant donc aux intervalles R₁-R'₁, R₂-R'₂ ...) pour affiner le diagnostic en cas de double détection avérée : si les valeurs correspondantes sont supérieures à une valeur limite, alors il s'agit vraisemblablement d'une double détection résultant d'une extrasystole ventriculaire (bigéminisme) ; dans le cas contraire, les intervalles RR' courts révèlent la détection d'un résidu de l'onde T malgré le filtrage.

## Revendications

1. Un dispositif médical implantable actif, notamment un stimulateur cardiaque, défibrillateur et/ou cardioverteur, comprenant :
- des moyens de détection des événements spontanés dans une cavité cardiaque, ventriculaire ou auriculaire,
- des moyens de mesure des intervalles séparant les événements successifs ainsi détectés,
- des moyens d'analyse du rythme cardiaque, opérant en fonction des valeurs d'intervalles ainsi mesurées, et
- des moyens d'élimination des doubles détections d'un même événement, lorsqu'un événement est suivi d'un artefact susceptible d'être également détecté,
dispositif **caractérisé en ce que** les moyens d'élimination des doubles détections comprennent des moyens aptes à évaluer des intervalles successifs mesurés (t₁, t₂, t₃, t₄, t₅, t₆, ...) séparant une série d'événements consécutifs détectés (R₁, R'₁, R₂, R'₂, R₃, R'₃, ...), à y identifier des intervalles courts et des intervalles longs, et considérer qu'il y a double détection en présence d'une alternance d'intervalles courts (t₁, t₃, t₅, ...) et d'intervalles longs (t₂, t₄, t₆, ...) dans les intervalles successifs.

2. Le dispositif de la revendication 1, dans lequel les moyens d'élimination des doubles détections comprennent des moyens d'analyse statistique de la distribution des valeurs desdits intervalles successifs.

3. Le dispositif de la revendication 2, dans lequel les moyens d'analyse statistique de la distribution comprennent :
- des moyens de classification, aptes à distribuer lesdits intervalles successifs en un histogramme comportant une pluralité de classes correspondant à des plages de valeurs consécutives d'intervalles, et
- des moyens pour déterminer, à partir du nombre d'intervalles classés dans chaque plage, la présence de deux pics statistiques équivalents dans l'histogramme et, dans ce cas, considérer qu'il y a eu double détection.

4. Le dispositif de la revendication 3, dans lequel les moyens d'analyse statistique de la distribution sont des moyens aptes à ne déterminer qu'il y a présence de deux pics équivalents que si ces pics sont des pics distincts.

5. Le dispositif de la revendication 4, dans lequel les moyens d'analyse statistique de la distribution sont des moyens aptes à considérer lesdits pics comme distincts s'il sont séparés par au moins une classe vide.

6. Le dispositif de la revendication 3, dans lequel les moyens d'analyse statistique de la distribution sont des moyens aptes à ne déterminer qu'il y a présence de deux pics équivalents que si le pic principal rassemble plus de 40 % du nombre total des intervalles.

7. Le dispositif de la revendication 3, dans lequel les moyens d'analyse statistique de la distribution sont des moyens aptes à ne déterminer qu'il y a présence de deux pics équivalents que si le pic secondaire rassemble autant d'intervalles que le pic principal.

8. Le dispositif de la revendication 1, dans lequel les moyens d'élimination des doubles détections comportent des moyens pour évaluer la durée C des cycles courts, la durée L des cycles longs et la durée M des cycles intermédiaires, et pour signaler aux moyens d'analyse du rythme cardiaque qu'il y a eu double détection si : L ≈ C + M.

## Patentansprüche

1. Aktive implantierbare medizinische Vorrichtung, insbesondere Herzschrittmacher, Defibrillator und/oder Kardioverter, umfassend:
- Mittel zur Erkennung von spontanen Ereignissen in einer Herzvorhof- oder ventrikulären Herzkammer,
- Mittel zur Messung von Intervallen, welche die so erkannten aufeinander folgenden Ereignisse trennen,
- Mittel zur Analyse des Herzrhythmus, welche abhängig von den so gemessenen Werten der Intervalle arbeiten, und
- Mittel zur Eliminierung von Zweifacherkennungen eines gleichen Ereignisses, wenn ein Ereignis von einem Artefakt gefolgt wird, das geeignet ist, gleichermaßen erkannt zu werden,
wobei die Vorrichtung **dadurch gekennzeichnet ist, dass** die Mittel zur Eliminierung von Zweifacherkennungen Mittel umfassen, die eingerichtet sind, gemessene, aufeinander folgende Intervalle (t₁, t₂, t₃, t₄, t₅, t₆, ...) auszuwerten, welche eine Reihe von erkannten, aufeinander folgenden Ereignissen (R₁, R'₁, R₂, R'₂, R₃, R'₃, ...) trennen, um dort kurze Intervalle und lange Intervalle zu identifizieren, und es zu betrachten, dass es eine zweifache Erkennung gibt bei Anwesenheit einer Alternierung von kurzen Intervallen (t₁, t₃, t₅, ...) und langen Intervallen (t₂, t₄, t₆....) in den aufeinander folgenden Intervallen.

2. Vorrichtung nach Anspruch 1, in welcher die Mittel zur Eliminierung von Zweifacherkennungen Mittel umfassen zur statistischen Analyse der Verteilung der Werte der aufeinander folgenden Intervalle.

3. Vorrichtung nach Anspruch 2, worin die Mittel zur statistischen Analyse der Verteilung umfassen:
- Mittel zur Klassifikation, eingerichtet, die aufeinander folgenden Intervalle in ein Histogramm zu verteilen, welches eine Vielzahl von Klassen umfasst, die Bereichen von Werten aufeinander folgender Intervalle entsprechen, und
- Mittel zur Bestimmung, ausgehend von der Anzahl von Intervallen, die in jedem Bereich klassifiziert sind, des Vorhandenseins von zwei statistisch gleichwertigen Spitzen in dem Histogramm und, in diesem Fall, es zu betrachten, dass es eine Zweifachcrkennung gegeben hat.

4. Vorrichtung nach Anspruch 3, in welcher die Mittel zur statistischen Analyse der Verteilung Mittel sind, die eingerichtet sind, das Vorhandensein von zwei gleichwertigen Spitzen nur zu bestimmen, falls diese Spitzen verschiedene Spitzen sind.

5. Vorrichtung nach Anspruch 4, in welcher die Mittel zur statistischen Analyse der Verteilung Mittel sind, die eingerichtet sind, die Spitzen als verschieden zu betrachten, falls sie durch wenigstens eine leere Klasse getrennt sind.

6. Vorrichtung nach Anspruch 3, in welcher die Mittel zur statistischen Analyse der Verteilung Mittel sind, die eingerichtet sind, ein Vorhandensein von zwei äquivalenten Spitzen nur zu bestimmen, falls die Hauptspitze mehr als 40% der gesamten Zahl von Intervallen versammelt.

7. Vorrichtung nach Anspruch 3, in welcher die Mittel zur statistischen Analyse der Verteilung Mittel sind, die eingerichtet sind, ein Vorhandensein von zwei äquivalenten Spitzen nur zu bestimmen, falls die Sekundärspitze ebenso viele Intervalle versammelt wie die Hauptspitze.

8. Vorrichtung nach Anspruch 1, in welcher die Mittel zur Eliminierung von Zweifacherkennungen Mittel umfassen zum Auswerten der Dauer C von kurzen Zyklen, der Dauer L von langen Zyklen und der Dauer M von dazwischen liegenden Zyklen, und zum Signalisieren an die Mittel zur Analyse des Herzrhythmus, dass es eine Zweifacherkennung gegeben hat, falls: L ≈ C + M.

## Claims

1. An active implantable medical device, in particular a cardiac pacemaker, defibrillator and/or cardioverter, comprising:
- means for detecting spontaneous events in a ventricular or atrial cardiac cavity,
- means for measuring intervals separating successive events thus detected,
- means for analyzing the cardiac rate, operating according to the interval values thus measured, and
- means for eliminating double detections of a same event, when an event is followed by.an artifact likely to be also detected,
said device being **characterised in that** the means for eliminating double detections comprises means for evaluating successive measured intervals (t₁, t₂, t₃, t₄, t₅, t₆, ...) separating a series of consecutive detected events (R₁, R'₁, R₂, R'₂, R₃, R'₃, ...), for identifying therefrom short intervals and long intervals, and decide that there is a double detection in the event of an alternation of short intervals (t₁, t₃, t₅, ...) and long intervals (t₂, t₄, t₆, ...) in the successive intervals.

2. The device of claim 1, wherein the means for eliminating double detections further comprises means for statistically analyzing the distribution of the values of aid successive intervals.

3. The device of claim 2, wherein the statistical analysis means comprises:
- classification means, for distributing said successive intervals in a histogram comprising a plurality of classes corresponding to ranges of consecutive values of intervals, and
- means for determining, from the number of intervals classified in each range, the presence of two equivalent statistical peaks in the histogram and, in such event, decide that there has been a double detection.

4. The device of claim 3, wherein the means for statistical analysis of the distribution is a means adapted to determine that there is a presence of two equivalent peaks only if said peaks are distinct peaks.

5. The device of claim 4, wherein the means for statistical analysis of the distribution is a means adapted to decide that said peaks are distinct if they are separated by at least one empty class.

6. The device of claim 3, wherein the means for statistical analysis of the distribution is a means adapted to determine that there is a presence of two equivalent peaks only if the main peak counts for more than 40% of the total number of the intervals.

7. The device of claim 3, wherein the means for statistical analysis of the distribution is a means adapted to determine that there is a presence of two equivalent peaks only if the secondary peak counts for as many intervals as the main peak.

8. The device of claim 1, wherein the means for eliminating double detections comprises means for evaluating the duration C of the short cycles, the duration L of the long cycles and the duration M of the intermediate cycles, and for signalling to the means for analyzing the cardiac rate that there has been a double detection if L ≈ C + M.
